(19) Europäisches Patentamt
European Patent Office
Office européen des brevets

(11) **EP 4 696 235 A1**

(12) **EUROPEAN PATENT APPLICATION**
published in accordance with Art. 153(4) EPC

(43) Date of publication:
**18.02.2026 Bulletin 2026/08**

(21) Application number: **24788605.4**

(22) Date of filing: **29.03.2024**

(51) International Patent Classification (IPC):
**A61B 5/151** (2006.01)    **G06T 7/00** (2017.01)

(52) Cooperative Patent Classification (CPC):
**A61B 5/151; G06T 7/00**

(86) International application number:
**PCT/JP2024/013271**

(87) International publication number:
**WO 2024/214578 (17.10.2024 Gazette 2024/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **11.04.2023 JP 2023064490**

(71) Applicant: Hitachi High-Tech Corporation
Minato-ku
Tokyo 105-6409 (JP)

(72) Inventors:
• **NAGASAKA, Akio**
**Tokyo 100-8280 (JP)**
• **KATO, Masahiro**
**Tokyo 105-6409 (JP)**
• **SUGIYAMA, Kimikazu**
**Tokyo 105-6409 (JP)**
• **IRIE, Takashi**
**Tokyo 105-6409 (JP)**

(74) Representative: **MERH-IP Matias Erny Reichl Hoffmann**
**Patentanwälte PartG mbB**
**Paul-Heyse-Straße 29**
**80336 München (DE)**

(54) **BLOOD COLLECTION DEVICE AND METHOD**

(57) A blood collection apparatus includes: a light source which emits light to a body part of a subject; a camera which captures an image of a blood vessel running subcutaneously in the body part from the same side or the opposite side of the light source; a movement mechanism which moves a relative position of the puncture needle to the body part; and a computation unit which calculates puncture suitability as a quantitative numerical value for each of points in a range in which the puncture needle is allowed to move on a captured image of the blood vessel captured by the camera, the puncture suitability being an indicator of whether the point is suitable for puncture or not. The computation unit is configured to select a location best suited for insertion of the puncture needle in the body part based on the puncture suitability calculated for each point in the range in which the puncture needle is allowed to move on the captured image, and to control the movement mechanism such that the puncture needle is inserted at the selected location.

FIG. 5

OPTIMAL PUNCTURE POINT EXTRACTION PROCESS

START

VESSEL ENHANCEMENT PROCESS — S10

VESSEL PATTERN EXTRACTION PROCESS — S11

VESSEL PATTERN THINNING PROCESS — S12

PER-POINT VESSEL ASSESSMENT VALUE CALCULATION PROCESS — S13

TOTAL ADJACENT VESSEL ASSESSMENT VALUE CALCULATION PROCESS — S14

MAXIMUM ASSESSMENT VALUE CALCULATION PROCESS — S15

END

EP 4 696 235 A1

## Description

[Technical Field]

The present invention relates to a blood collection apparatus and method, and is advantageously applied to, for example, an automatic blood collection apparatus that performs blood collection in an automated manner.

[Background Art]

Blood tests are essential for obtaining information as basis for diagnosing different diseases in medicine. Recently, it has also been expected that blood tests are utilized in early diagnosis of disease signs in the interest of curbing of ever-growing medical costs, and demand for blood tests will increase even more in the future.

With such a growing demand, a concern is that burden on medical practitioners who perform blood collection becomes heavier. With such a background, there has been growing interest recently in automatic blood collection apparatuses that perform blood collection in an automated manner without human intervention. There is particularly need for compact and space-saving automatic blood collection apparatuses that can be installed without taking out too much of limited space in medical settings.

Known automatic blood collection apparatuses of this type include a blood collection apparatus disclosed in Patent Literature 1. The blood collection apparatus is such that when a subject presents his/her fingertip at a predetermined position on the apparatus, a needle automatically moves and punctures the fingertip to create a small wound, and blood flowing from it is received into a blood collection container to collect blood. This blood collection apparatus achieves downsizing of the apparatus by use of a fingertip as the site of blood collection.

[Citation List]

[Patent Literature]

[Patent Literature 1]
Japanese Patent No. 6994910

[Non Patent Literature]

[Non Patent Literature 1]
Li, X., Lin, J., Pang, Y., Huang, L., Zhong, L., Li, Z., "Fingertip Blood Collection Point Localization Research Based on Infrared Finger Vein Image Segmentation", IEEE Transactions on Instrumentation and Measurement, Vol.71, pp.1-12 (2021)

[Summary of Invention]

[Technical Problem]

In general, a person's finger has parts suitable for blood collection and parts not suitable. Patent Literature 1, however, gives no consideration to the suitability of a blood collection point, only disclosing that the blood collection apparatus mechanically inserts a puncture needle at a fixed point. Thus, it has the challenge of possibly failing to collect a sufficient amount of blood due to individual difference of the subject's finger, the posture of presentation, or change in position.

Meanwhile, studies aiming at stable automated blood collection include a study described in Non Patent Literature 1, for example. Practically, Non Patent Literature 1 discloses a method that captures an image of a vessel pattern in a finger with a near-infrared camera, performs analysis processing on the captured image, and extracts a vessel branch point as the location of puncture.

When a medical practitioner manually performs blood collection, it is also a common practice to insert a blood collection puncture needle aiming at a blood vessel in an arm, and a vessel branch point in particular is expected to have a relatively high blood flow because it is a site where multiple blood vessels meet. Accordingly, stable blood collection is sought by enabling control with a robot arm so that a blood collection puncture needle can puncture a finger at a certain location and programming the control such that the blood collection puncture needle will puncture such a vessel branch point.

However, a finger commonly has a number of vessel branch points therein, and Non Patent Literature 1 does not disclose or suggest the way of prioritizing them, such as how to select one of them as the puncture site. The technique thus has the disadvantage of eventually requiring human determination.

The present invention has been made in view of the foregoing and an object thereof is to provide a blood collection apparatus and method that can perform stable blood collection without human intervention.

[Solution to Problem]

In order to attain the object, the present invention provides a blood collection apparatus that performs blood collection by inserting a puncture needle into a body part presented by a subject, the blood collection apparatus including: a light source which emits light to the body part; a camera which captures an image of a blood vessel running subcutaneously in the body part from the same side or the opposite side of the light source; a movement mechanism which moves a relative position of the puncture needle to the body part; and a computation unit which calculates puncture suitability as a quantitative numerical value for each of points in a range in which the puncture needle is allowed to move on a captured image of the blood vessel captured by the camera, the puncture suitability being an indicator of whether the point is suitable for puncture or not. The computation unit selects a location best suited for insertion of the puncture needle in the body part based on the puncture suitability calculated for each point in the range in which the puncture needle is allowed to move on the captured image, and controls the movement mechanism such that the puncture needle is inserted at the selected location.

The present invention also provides a method of blood collection performed by a blood collection apparatus that performs blood collection by inserting a puncture needle into a body part presented by a subject, the blood collection apparatus including: a light source which emits light to the body part; a camera which captures an image of a blood vessel running subcutaneously in the body part from the same side or the opposite side of the light source; a movement mechanism which moves a relative position of the puncture needle to the body part; and a computation unit which calculates puncture suitability as a quantitative numerical value for each of points in a range in which the puncture needle is allowed to move on a captured image of the blood vessel captured by the camera, the puncture suitability being an indicator of whether the point is suitable for puncture or not. The method includes a first step of, by the computation unit, selecting a location best suited for insertion of the puncture needle in the body part based on the puncture suitability calculated for each point in the range in which the puncture needle is allowed to move on the captured image, and a second step of, by the computation unit, controlling the movement mechanism such that the puncture needle is inserted at the selected location.

The blood collection apparatus and method according to the present invention can select a location best suited for insertion of a puncture needle based on the puncture suitability.

[Advantageous Effect of Invention]

According to the present invention, stable blood collection can be performed irrespective of the subject's posture of presenting a body part, the condition of the body part, and individual differences in shape or the like.

[Brief Description of Drawings]

[Figure 1] Figure 1 shows a general configuration of an automatic blood collection apparatus according to first to fourth embodiments.
[Figure 2] Figure 2 is a top view showing an exemplary configuration of a portion of a movement mechanism.
[Figure 3] Figure 3 shows an example of a captured image.
[Figure 4] Figure 4 is a flowchart illustrating a processing procedure of an automatic blood collection process.
[Figure 5] Figure 5 is a flowchart illustrating a processing procedure of an optimal puncture point extraction process according to the first embodiment.
[Figure 6] Figure 6 is a diagram representing processing actions in a vessel enhancement process.
[Figure 7] Figure 7 shows how a captured image changes through an optimal puncture point extraction process.
[Figure 8] Figure 8 shows a characteristic curve diagram for use in describing how a vessel assessment value is calculated.
[Figure 9] Figure 9 is an illustration for use in describing a total adjacent vessel assessment value calculation process.
[Figure 10] Figure 10 is a flowchart illustrating a processing procedure of the optimal puncture point extraction process according to a second embodiment.
[Figure 11] Figure 11 is an illustration for use in describing the second embodiment.
[Figure 12] Figure 12 is a flowchart illustrating a processing procedure of the optimal puncture point extraction process according to the second embodiment.
[Figure 13] Figure 13 is an illustration for use in describing a third embodiment.

[Figure 14] Figure 14 is a perspective view schematically showing an overall configuration of an automatic blood collection system according to a fourth embodiment.

[Description of Embodiments]

An embodiment of the present invention will be described in detail with reference to the drawings.

(1) First embodiment

(1-1) Puncture suitability

Puncture suitability is described first. Since the position of a vessel branch point in a finger is completely different from person to person, it cannot be ensured whether a branch point exists within a range in which a needle is allowed to move and puncture. A small-sized blood collection apparatus in particular, such as disclosed in Patent Literature 1, requires downsizing of a mechanism for moving the needle as well, which inevitably limits the freedom of mobility.

Thus, instead of basing on information on a feature with which no assessment can be made if it does not exist in a range of interest, such as a vessel branch point, it is desirable to determine a quantitative numerical indicator adapted to an expectation of the amount of blood collected for a certain point in a range of interest and to enable selection of a point suitable for puncture according to the relative magnitude of the indicator. This allows for selection of the second best location within the range of interest even when a location suitable for puncture is not present in the range and can avoid a significant decrease in the amount of blood collection. Thus, stability of blood collection can be increased even with a small-sized blood collection apparatus. Such a numerical indicator is called puncture suitability below.

In calculation of the puncture suitability, it is necessary to numerically reflect how much amount of blood collection can be expected. Since the amount of blood collected is considered to be generally proportional to the blood volume at the location of puncture, being a point on a blood vessel in which blood densely concentrates in a body part of interest is a basic condition for the location of puncture. The puncture suitability then needs to be such an indicator that gives high assessment to a portion of blood vessels present in the body part of interest that contains the most blood.

For measurement of the distribution of the blood volume in the body part of interest, near-infrared measurement, which is also used in Non Patent Literature 1 cited above, can be used. When near-infrared light is transmitted through a living body and captured with a camera enabled for near-infrared wavelength bands, blood vessels are captured as dark lines. This is because hemoglobin contained in blood has a property of absorbing near-infrared light and transmission of near-infrared light causes the light to be weakened when passing through blood vessels with much hemoglobin, resulting in the blood vessels being captured relatively dark compared to the surroundings. A captured image of blood vessels thus obtained indicates that portions with darker lines have more blood; and basically, by analyzing such a captured image, the puncture suitability for each point on a blood vessel can be calculated.

However, images of blood vessels captured with infrared light are typically unclear, and easiness of light transmission is prone to unevenness due to not only blood volume but also difference in tissue composition in a living body and the like. In determination of the puncture suitability at a certain point, it is difficult to accurately determine only from a local feature, like low brightness at the point, whether a high volume of blood is actually present there or the feature coincidentally occurred due to unevenness. Inaccurate determination leads to inability to secure a stable amount of blood, so it is essential to have means that can robustly detect a portion with high blood volume. Thus, in selecting a point for puncture (which is hereinafter called a puncture point), it is necessary to set and use a puncture suitability with suppressed influence of local variations by reflecting not only a local feature at a single candidate point but the distribution state of blood volume over a wider range including a vicinity of the point.

An automatic blood collection apparatus according to the first embodiment described below selects the best suited puncture point (which is called an optimal puncture point hereinafter) in a body part presented by the subject in consideration of such puncture suitability and performs blood collection by inserting a puncture needle at the selected puncture point.

(1-2) Configuration of the automatic blood collection apparatus in the first embodiment

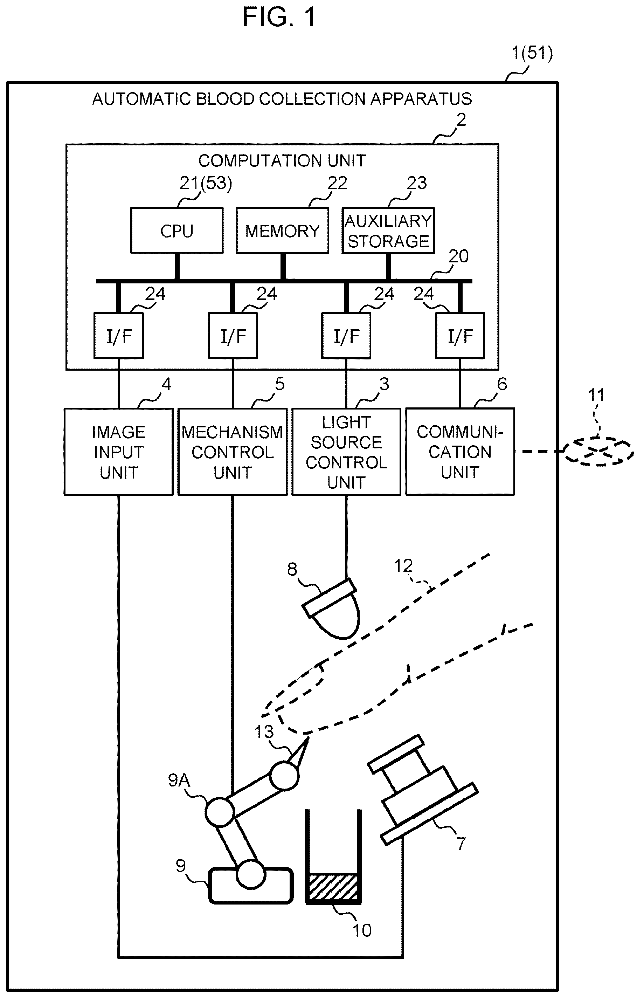

In Figure 1, the automatic blood collection apparatus according to this embodiment is generally shown at 1. The automatic blood collection apparatus 1 includes a computation unit 2, a light source control unit 3, an image input unit 4, a mechanism control unit 5 and a communication unit 6, a camera 7, a near-infrared light source 8, a movement mechanism 9, and a blood collection tube 10.

The computation unit 2 includes a CPU (Central Processing Unit) 21, a memory 22, an auxiliary storage 23, and interfaces 24 that are provided for the light source control unit 3, the image input unit 4, the mechanism control unit 5 and the communication unit 6, respectively. The CPU 21, the memory 22, the auxiliary storage 23 and the interfaces 24 are coupled to each other by an internal bus 20. The CPU 21, the memory 22, the auxiliary storage 23 and the interfaces 24 can also be coupled to each other by a dedicated bus such as for the purpose of acceleration.

The CPU 21 is a processor responsible for controlling the operation of the entire automatic blood collection apparatus 1. The memory 22 is constructed of RAM (Random Access Memory) and ROM (Read Only Memory) and used for permanently storing programs necessary for processing or temporarily storing data necessary for processing. By the CPU 21 executing programs stored in the memory 22, various kinds of processing are executed in the automatic blood collection apparatus 1 as a whole, as discussed later.

The auxiliary storage 23, which is constructed of flash memory and a hard disk device, for example, is used for storing data that needs to be stored permanently. Input and output of data to and from the auxiliary storage 23 takes place via the memory 22 under the control of the CPU 21.

The interfaces 24 are devices with the function of coupling between the computation unit 2 and the corresponding functional blocks (the light source control unit 3, the image input unit 4, the mechanism control unit 5, and the communication unit 6) so that necessary data can be exchanged between the computation unit 2 and the functional blocks.

The presence of these interfaces allows the computation unit 2 to control the light emitting status of the near-infrared light source 8 via the light source control unit 3, to input an output image from the camera 7 via the image input unit 4, and to control and drive the movement mechanism 9 via the mechanism control unit 5. The computation unit 2 can also perform communications with external devices (not shown) coupled to a network 11 via the communication unit 6. Such communications allow for sharing part or all of processing associated with blood collection with an external device (e.g., an external computing device as host) or cooperating with various services on clouds, for example.

The camera 7 is constructed of a CCD (Charge Coupled Device) image sensor, a CMOS (Complementary Metal-Oxide Semiconductor) image sensor, for example, and captures an image of a subcutaneous vessel pattern in a body part (hereinafter a finger) 12 of the subject at which blood collection is to be performed, and outputs image data for the captured image to the image input unit 4.

The near-infrared light source 8 is constructed of a near-infrared LED (Light Emitting Diode), for example, and based on driving electric power fed from the light source control unit 3, it emits near-infrared light of an intensity dependent on the electric energy of the driving electric power. The near-infrared light source is fixedly disposed such that its optical axis aligns with the optical axis of the camera 7 across a distance enough for allowing insertion of the finger 12 of the subject, as shown in Figure 1, for example. The reason for placing the subject's finger 12 between the near-infrared light source 8 and the camera 7 is that a human finger is thick enough for allowing sufficient transmission of near-infrared light, and vessel patterns with higher contrast can be acquired when the finger is captured by passing near-infrared light through it from the opposite side of the finger surface facing the camera 7.

It is also possible to place the light source on the same side as the camera 7, emit light onto the target surface of the finger 12 being captured, and capture vessel patterns with light reflected back from the finger. In this case, however, reflection of light on the skin surface of the finger 12 is strong and light reflected at a blood vessel deep in the finger will be relatively weakened, thus resulting in vessel patterns with low contrast. Since such a reflected-light approach allows the camera 7 and the light source to be compactly accommodated on the same side, it has the advantage of keeping the size of an imaging-related system compact and is suitable when downsizing of the device is a high priority. For cases where blood collection is performed on a thick body part such as an arm, the reflected light approach is also more appropriate because an approach based on light transmission would cause light attenuation in the body part and a sufficient amount of light could not pass through, failing to provide contrast of vessel patterns.

The movement mechanism 9 is a mechanism to move a relative position of the puncture needle 13 to the finger 12, including the position in the direction of inserting the puncture needle 13 into the finger 12 presented by the subject at a predetermined location in a predetermined state and in the direction of pulling out the puncture needle 13 inserted into the finger 12 from the finger 12. The movement mechanism 9 includes an ejection mechanism 9A with the puncture needle 13 attached at a tip thereof in a replaceable manner. The ejection mechanism 9A includes an actuator such as a motor, not shown, with which the puncture needle 13 can be moved in the direction of inserting it into the subject's finger 12 and the direction of pulling out the inserted puncture needle.

The light source control unit 3 is a functional block with the function of controlling the luminosity of the near-infrared light emitted by the near-infrared light source 8 so that vessel patterns are clearest in an image captured by the camera 7, based on a target value designated by a program executed by the CPU 21. Specifically, using PWM (Pulse Width Modulation), the light source control unit 3 controls driving electric energy applied to near-infrared light source 8 at a target value designated by the program executed by the CPU 21.

The image input unit 4 is a functional block with the function of converting aforementioned image data provided from the camera 7 into a format manageable at the computation unit 2 and storing the converted image data in the memory 22. The mechanism control unit 5 controls the movement mechanism 9 in response to an instruction from the program executed by the CPU 21.

In the automatic blood collection apparatus 1 configured as described above, the CPU 21 analyzes a captured image of vessel patterns acquired as discussed above based on a program stored in the memory 22, and determines the coordinate values of the point determined to be best suited for puncture on the captured image (the optimal puncture point). The CPU 21 then provides the coordinate values of the optimal puncture point determined to the mechanism control unit 5.

When the coordinate values of the optimal puncture point are provided from the CPU 21, the mechanism control unit 5 controls the movement mechanism 9 to insert the puncture needle 13 into the finger 12 presented by the subject at the coordinate values. Prior to this, the mechanism control unit 5 performs calibration in advance such that certain coordinates on the finger 12 shown on a captured image from the camera 7 coincide with the actual point on the finger at which the puncture needle 13 is inserted, and stores the correspondence between them.

Upon insertion of the puncture needle 13 into the finger 12 of the subject, the mechanism control unit 5 operates the movement mechanism 9 to move the puncture needle 13 in the direction of pulling it out from the finger 12 of the subject, and then controls the movement mechanism 9 so that the blood collection tube 10 is moved immediately below the location of insertion of the puncture needle 13 in the finger 12 of the subject. As a result, blood flowing from the finger 12 of the subject is received in the blood collection tube, and blood collection is complete when a prescribed volume of blood has been collected.

It is also possible to employ a thin, tubular needle similar to an injection needle as the puncture needle 13 and connect the puncture needle 13 and a blood collection tube with a tube and the like and to wait for a prescribed volume of blood to be collected in the blood collection tube 10 while leaving the puncture needle 13 inserted in the finger 12 of the subject.

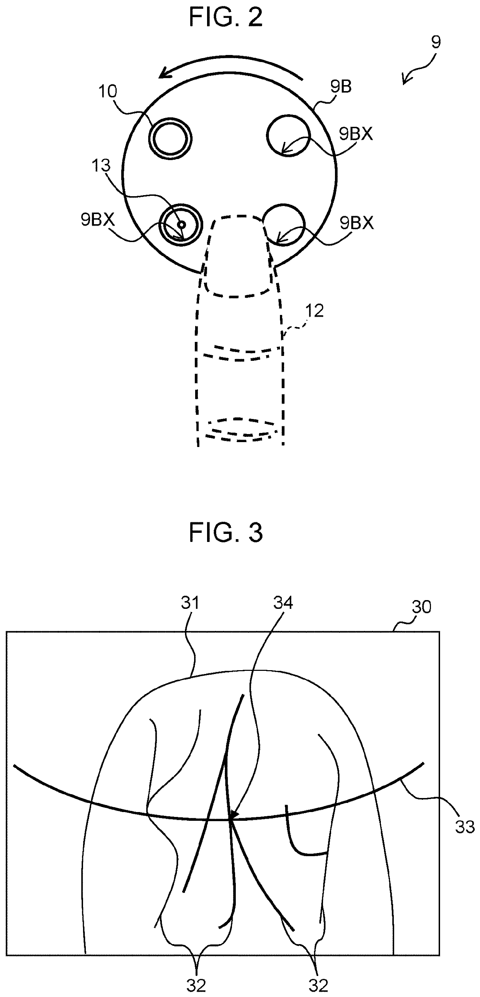

Figure 2 shows an exemplary configuration of a portion of the movement mechanism 9. The movement mechanism 9 includes a turntable 9B that is rotated by a motor, and the ejection mechanism 9A (Figure 1), which pushes the puncture needle 13 from an upper surface of the turntable 9B toward the finger 12 of the subject presented at a predetermined location.

The turntable 9B is provided with multiple holes (four in Figure 2) on the same circumferential track. The puncture needle 13 is positioned so that it is pushed toward the subject's finger from at least one hole 9BX of the holes 9BX, and the blood collection tube 10 is set by being fit into another hole 9BX neighboring that hole 9BX.

At the time of blood collection, in the movement mechanism 9, the turntable 9B is rotated so that the puncture needle 13 is located immediately below the subject's finger 12 presented at a predetermined location in a predetermined state, and then the puncture needle 13 is pushed out of the hole 9BX in the turntable 9B by the ejection mechanism 9A and inserted at the optimal puncture point on the subject's finger 12 under the control of the mechanism control unit 5 (Figure 1). Subsequently, the puncture needle 13 is returned into the hole 9BX in the turntable 9B by the ejection mechanism 9A, and then the turntable 9B is rotated so that the blood collection tube 10 is located immediately below the finger 12. As a result, blood flowing from the finger is collected in the blood collection tube 10.

Such a movement mechanism 9 in this embodiment allows blood collection to be performed just by having two degrees of freedom: rotation of the turntable 9B and movement of the puncture needle 13 in the inserting direction and the reverse direction, and has an advantage of allowing consumables that are used only once per blood collection, such as the puncture needle 13 and the blood collection tube 10, to be easily managed (such as replaced) from the upper side of the turntable 9B together. The movement mechanism 9 in this embodiment can also be compact in size due to a simple structure, hence contributing to downsizing of the entire automatic blood collection apparatus 1.

Figure 3 shows an example of a captured image 30 of the subject's finger 12 taken by the camera 7 of the automatic blood collection apparatus 1 in this embodiment. In this captured image 30, a view 31 of the subject's finger 12 is shown in a center portion of the image, with vessel patterns 32 in the finger 12 appearing inside the view 31. Points on these vessel patterns 32 represent portions with relatively high blood volume.

An arc track 33 in the figure represents a movement range of the puncture needle 13. The optimal puncture point needs to be extracted from points on the arc track 33, and among others, an intersection 34 with a blood vessel that is estimated to have high blood volume will be selected. Since the vessel patterns 32 are generally formed like a network, the arc track 33 and the vessel patterns 32 typically intersect at multiple points. Accordingly, among such intersections 34, one that is estimated to have the highest blood volume needs to be extracted and determined as the point to actually insert the puncture needle 13.

Although the movement range of the puncture needle 13 is described as being an arc track in this embodiment, the movement mechanism 9 for moving the puncture needle 13 is not limited to the configuration shown in Figure 2. Depending on the configuration of the movement mechanism 9 applied, the movement range of puncture needle 13 varies as well. Increasing the degree of freedom in the movement the puncture needle 13 expands the movement range of the puncture needle 13 as well, also allowing for a strip range with a width, rather than a line as in Figure 3. Also, the movement range of the puncture needle 13 needs not to be arc-shaped, but may be linear, rectangular, or fan-shaped. In the following, however, the movement range of the puncture needle 13 on the captured image 30 is assumed to be horizontally linear for the sake of simplicity.

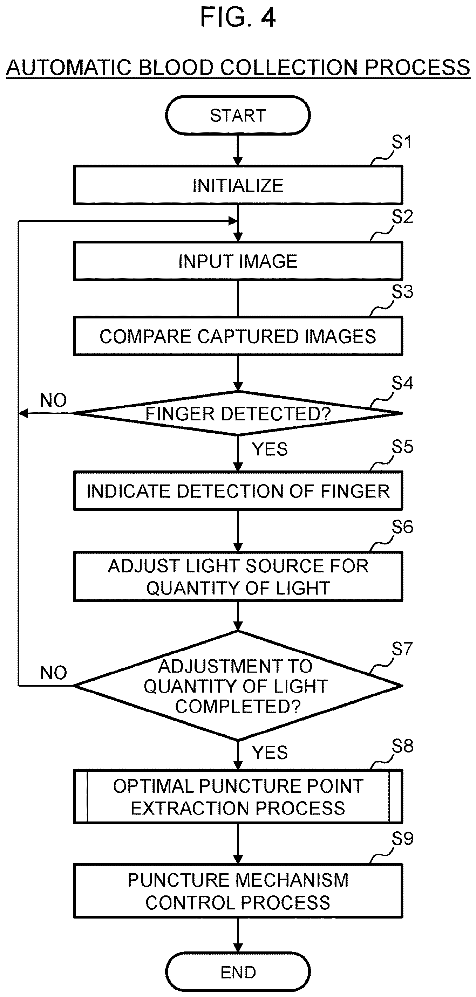

Figure 4 shows a flow of a sequential process performed by the CPU 21 (Figure 1) of the computation unit 2 of the automatic blood collection apparatus 1 (which is hereinafter called an automatic blood collection process) during blood collection. This automatic blood collection process is started in response to a predetermined operation performed on the automatic blood collection apparatus 1 by the subject or an attendant, for example.

The CPU 21 first initializes various kinds of processing to be executed and hardware (S1). The CPU 21 also activates the camera 7 (Figure 1) to start capturing images, and makes image data for the resulting captured image to be taken into the image input unit 4 (Figure 1) (S2). The image data taken at this point is stored in a predetermined area in the memory 22 (Figure 1) of the computation unit 2 by the image input unit 4.

The CPU 21 proceeds to compare the captured image based on the last image data that was stored in the memory 22 with the captured image based on the current image data stored in the memory 22 in step S2 (S3), and determines whether a large image change (an image change above a predefined threshold value) that is caused by presentation of a body part for blood collection (here, a finger) at a predetermined location in a predetermined state has been detected or not (S4).

Receiving a negative result in this determination means that the subject has not presented the finger 12 (Figure 1) at the predetermined location in the predetermined state yet. So, the CPU 21 returns to step S2 and repeats the processing at steps S2 to S4 until an affirmative result is obtained in step S4.

When an affirmative result is subsequently obtained in step S4 in response to presentation of the subject's finger 12 at the predetermined location in the predetermined state, the CPU 21 notifies the subject or the attendant that blood collection is about to start, if necessary (S5). For example, the CPU 21 notifies the subject or the attendant that blood collection is about to start in a manner optimal for the environment of application, such as displaying text or an icon on a display to the effect that the subject's finger 12 has been detected or providing an audible announcement or alarm sound. The CPU 21 may also use a dedicated sensor to detect whether the subject's finger 12 has been presented at a predetermined location in a predetermined state or not. This processing at step S5 may also be omitted.

Next, the CPU 21 gives an instruction to the light source control unit 3 (Figure 1) so that the near-infrared light source 8 (Figure 1) starts illumination, and adjusts the quantity of emitted light of the near-infrared light source 8 via the light source control unit 3 so as to create a condition where the subject's finger 12 is captured under appropriate luminosity (S6). Whether the subject's finger 12 is under appropriate luminosity or not can be determined by analyzing the captured image that is based on the image data stored in the memory 22 and determining if an average brightness of the view 31 (Figure 3) of the finger 12 is within a predefined range or not.

The CPU 21 further determines whether adjustment to the quantity of emitted light of the near-infrared light source 8 such that the subject's finger 12 is under appropriate luminosity is complete or not (S7), and when it obtained a negative result, it returns to step S2, after which it repeats the processing at steps S2 to S7 until an affirmative result is obtained in step S7.

When the CPU 21 obtains an affirmative result in step S7 in response to completion of the adjustment to the quantity of emitted light of the near-infrared light source 8, it performs an optimal puncture point extraction process to extract the optimal puncture point from the region of the view 31 of the finger 12 shown in the captured image (S8). After extracting the optimal puncture point in the optimal puncture point extraction process, the CPU 21 controls the movement mechanism 9 (Figure 1) via the mechanism control unit 5 (Figure 1) so that the puncture needle 13 is moved to the extracted optimal puncture point and the puncture needle 13 is inserted at optimal puncture point (S9).

As a result, the puncture needle is inserted at the optimal puncture point and blood collection is started, after which the blood collection is complete when a necessary amount of blood has been collected. As to whether a necessary amount of blood has been collected or not, it may be determined that a necessary amount of blood has been collected by monitoring the weight of the blood collection tube 10 (Figure 1) and determining when the weight of the blood collection tube 10 has reached or exceeded a certain weight or by monitoring the level of blood in the blood collection tube 10 such as with a sensor and determining when the level has reached or exceeded a certain level. When a necessary amount of blood has been collected, the CPU 21 terminates the automatic blood collection process.

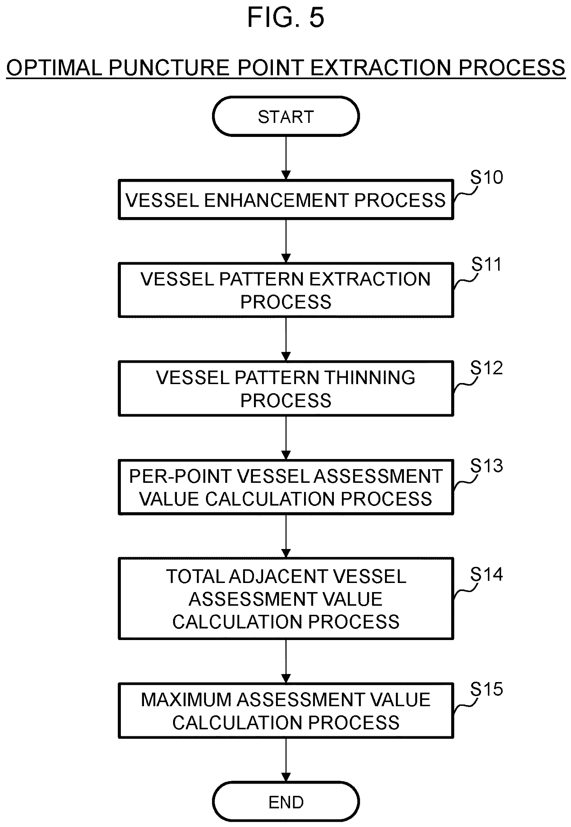

Figure 5 shows specific actions of the optimal puncture point extraction process, performed by the CPU 21 in step S8 in the automatic blood collection process described above. When it proceeds to step S8 in the automatic blood collection process, the CPU 21 starts the optimal puncture point extraction process shown in Figure 5.

The CPU 21 first performs vessel enhancement processing for enhancing blood vessels on the captured image currently stored in the memory 22 that was obtained at the most recent step S2 (S10). This is because a puncture point is essentially a point on a blood vessel, so accuracy of analysis can be increased by performing amplification processing so that vessel features become clearer prior to analysis processing.

Thus, the CPU 21 basically performs vessel enhancement processing on the basis of a common technique for edge extraction called difference of Gaussian. As it is a technique that amplifies and enhances a local change in brightness, the difference of Gaussian enhances not only contours but lines like blood vessels. It is also possible to suppress amplification of fine noise and stably enhance relatively thick blood vessels by using the difference of Gaussian to take a difference between two images (which are called image $G_1$ and image $G_2$ below) generated from the same image and being different in a smoothing parameter (the radius of a region referenced in smoothing).

As the surface of living body such as a finger has irregularities as represented by fingerprints, and additionally due to the effect of cornification caused by damaged skin, a captured image of blood vessels is prone to fine noise. With setting an appropriate smoothing level in the difference of Gaussian, such noise will be removed during smoothing and only the remaining stable blood vessels will be enhanced. By multiplying the pixel value of each pixel in the differential image ($G_1$ - $G_2$) between the image $G_1$ and the image $G_2$ by a factor of N and adding a constant C to the pixel value of each pixel, a vessel enhanced image that has a higher amplification rate for a greater value of N can be obtained. The constant C here is a correction term for avoiding a pixel value taking a negative value due to the difference. When the captured image is an image of 256 gray levels, for example, the median value, or 128, will be set as the constant C.

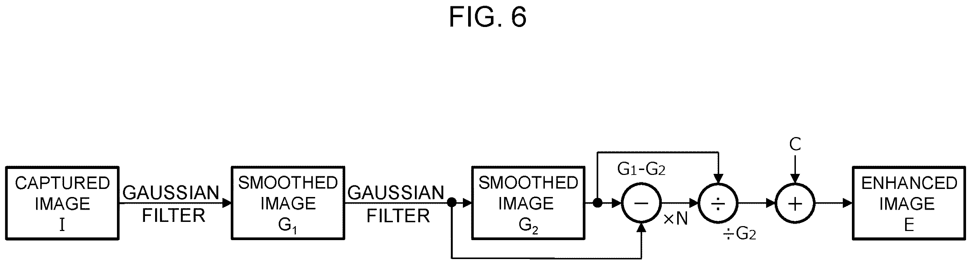

Figure 6 shows an example of a specific diagram of such vessel enhancement processing. In this example, smoothing processing (Gaussian filtering) is applied to a captured image I with its image data stored in the memory 22 and further smoothing processing (Gaussian filtering) is applied to a resulting first smoothed image $G_1$, thus generating a second smoothed image $G_2$. This is done for further suppressing the effect of noise by first generating images with noise other than blood vessels removed by the first smoothing processing and performing differential processing only with the images without noise, although the basic idea of using the difference between images with different smoothing parameters is similar to the above-mentioned flow.

At the same time, when calculating the puncture suitability discussed above, it is desirable that the thickness and/or density of blood vessels can be measured by the same criterion wherever they are located in a finger region. For example, if the brightness varies from location to location although the blood vessel is originally of the same density, the puncture suitability determined is likely to be a location-dependent unstable indicator. A near-infrared image of a body part in particular is prone to mild unevenness in light intensity due to the relation between the positioning of the light source and the location of the body part presentation or variations in composition in biological tissue.

Accordingly, in order to correct such unevenness, normalization processing is added to the course of the vessel enhancement process. Specifically, normalization is performed by multiplying the pixel value of each pixel in the difference image ($G_1$ - $G_2$) between the first smoothed image $G_1$ and the second smoothed image $G_2$ by a factor of N and further dividing the pixel value of each pixel by the pixel value of the corresponding pixel in the second smoothed image $G_2$, which has higher level of smoothness. Having high level of smoothness means a state where local features are lost and only more global features are left. Since most of light unevenness is global features, normalization can be performed with such a division. Such a way of normalization is simple yet is expected to provide a certain level of effect, but other ways of normalization may be employed when light unevenness should be removed more stably such as through device configuration.

Furthermore, in the vessel enhancement process, the aforementioned constant C is added to the pixel value of each pixel of the normalized image. This can provide an image with enhanced blood vessels (which is hereinafter called a vessel enhanced image) E.

The CPU 21 next performs a vessel pattern extraction process to extract vessel patterns 32 (Figure 3) from the vessel enhanced image resulting from the vessel enhancement processing (S11). While various existing techniques used in vein authentication, one of biometric authentication techniques, can be used for extraction of the vessel patterns 32, it is advantageous to use a technique that is capable of uniform extraction irrespective of the density or thickness of blood vessels, such as one based on the maximum curvature change rate. The vessel assessment to be discussed later uses the density and/or thickness of blood vessels as features, where extraction of vessel patterns and assessment of blood vessels can be processed in different stages to increase their respective processing accuracies and improve the accuracy of extraction of the final optimal puncture point by processing.

After this, the CPU 21 performs thinning processing to generate a pattern image including only center lines not dependent on the vessel thickness, from the vessel enhanced image to which the vessel pattern extraction process has been applied (S12). Such thinning of vessel patterns is performed in order to prevent excessive increase in the amount of processing that would occur if the thicknesses of blood vessels are maintained, because unnecessarily many pixels are involved per blood vessel in a subsequent vessel assessment.

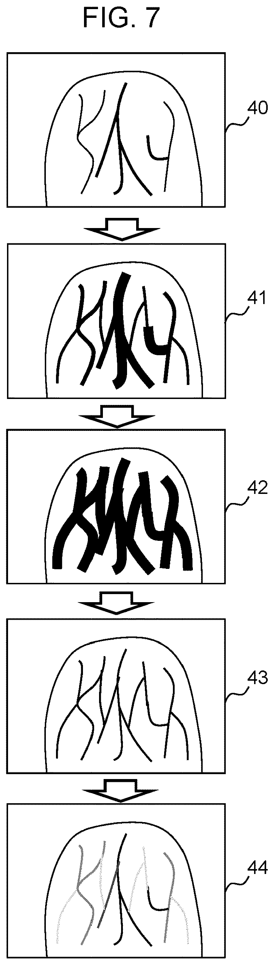

Figure 7 schematically shows how a captured image changes through the processing in steps S10 to S12 discussed above. At the top of Figure 7 is a captured image 40 taken by the camera 7, and this captured image 40 is converted into a vessel enhanced image 41 with generally increased contrast of blood vessels via the vessel enhancement process of step S10. This vessel enhancement processing makes obscure thin blood vessels clear and also suppresses noise arising from the surface state of living body.

By applying the vessel pattern extraction process of step S11 to the vessel enhanced image 41, a vessel pattern extracted image 42 with vessel patterns extracted uniformly irrespective of their thickness or density is acquired. The uniform extraction of vessel patterns enables binarization into blood vessels and others.

By further applying the vessel pattern thinning process of step S12 to the vessel pattern extracted image 42, a thinned image 43 representing the vessel patterns with a minimum number of pixels is obtained. The thinned image 43 is used as auxiliary information for determining whether a point is a point on a blood vessel, as a precondition for determining the optimal puncture point. As such, in the vessel pattern extraction process at step S11, any portions that can be blood vessels should be extracted with loose conditions for extraction, minimizing omissions as much as possible. A lighter and faster technique may be used if it can sufficiently reduce extraction omissions with loosened conditions, since over-detection is relatively allowable.

Returning to the description of Figure 5, the CPU 21 next performs a per-point vessel assessment value calculation process (S13), which calculates an assessment value as a blood vessel (which is called a vessel assessment value hereinafter) for each of points (pixels) in the thinned image 43 extracted as vessel patterns in the processing at steps S10 to S12. Since the vessel assessment value is used as an indicator for estimating the magnitude of blood volume, it focuses on the thickness of a blood vessel, which can be quantitatively assessed from a captured image of blood vessels.

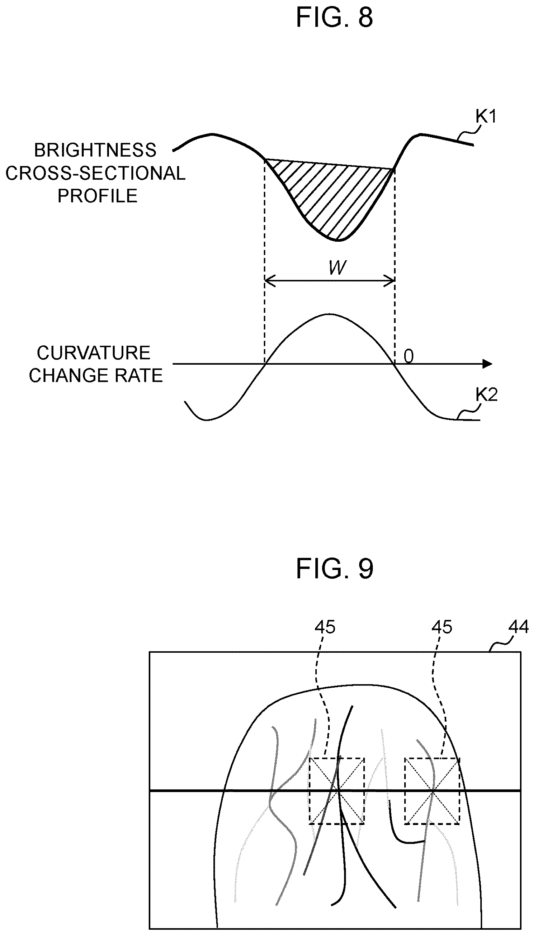

With Figure 8, an example of how to calculate a vessel assessment value is described. In Figure 8, a curve K1 indicates an example of a brightness cross-sectional profile around a blood vessel in a vessel enhanced image, and a curve K2 indicates the corresponding curvature change rate. The curvature change rate is representative of a brightness change rate at each point on the brightness cross-sectional profile. It assumes the maximum positive value at the peak with the lowest brightness and conversely assumes the minimum negative value at the peak with the highest brightness.

Here, when the width is $\delta$, curvature change rate $C(x)$ at point $P(x)$ of the x-coordinate on the brightness cross-sectional profile can be represented by:

[Expression 1]

$$C(x) = \{P(x + \delta, y) - P(x,y)\} + \{P(x - \delta, y) - P(x,y)\} \ldots$$
$$(1)$$

Using this curvature change rate $C(x)$, a vessel range in the vessel enhanced image 41 (Figure 7) can be determined. In the vessel enhanced image 41, portions that appear to be dark as blood vessels correspond to portions at which the curvature change rate $C(x)$ is generally positive. A vessel range can be extracted by detecting points at which the sign of curvature change rate $C(x)$ reverses and determining a segment of length W (Figure 8) of the portion lying between these points, in which a positive value continues. This length W will be called vessel width W below.

In the brightness cross-sectional profile, then an area located below the line connecting the brightnesses at both ends of the segment of the vessel width W serves as an overall indicator that reflects the thickness and density of a blood vessel at the same time, and this is utilized as a vessel assessment value. This vessel assessment value serves the initial purpose because it indicates a greater value as the vessel width W is larger and as the blood vessel is denser, that is, a fall in brightness from a blood vessel boundary is larger.

Such a vessel assessment value is determined for each point on a thinned vessel pattern in the thinned image 43 (Figure 7), and a vessel assessment value image 44 (Figure 7) with the pixel value of each point on a vessel pattern replaced with a vessel assessment value is generated in the following procedure. First, all of the pixels of the thinned image 43 are scanned and it is determined whether each pixel is a point on a thinned vessel pattern or not. For a pixel determined to be a point on a vessel pattern, a brightness cross-sectional profile centered at the pixel is taken from the vessel enhanced image 41 and the vessel assessment value at the pixel (point) is determined in the above-described manner.

However, a vessel assessment value can only be determined from a brightness cross-sectional profile that traverses a blood vessel in the width direction. For example, from a cross-sectional profile that is taken from an image horizontally, only assessment values related to blood vessels that run vertically on the image would be obtained. For a blood vessel that runs horizontally, only a profile scanning inside the blood vessel is obtained and there is no information for the width direction, so that its thickness and density cannot be measured.

Accordingly, vessel assessment values are determined with brightness cross-sectional profiles in two directions, i.e., the vertical and horizontal directions, respectively, and one of the two vessel assessment values that was calculated with the profile in a direction closer to the width direction of the blood vessel is used as the final assessment value at that pixel (point). As to determination of which direction is closer to the width direction of the blood vessel, the curvature change rates of brightness in different scanning directions at the pixel (point) are compared to each other and a direction that shows a greater value is assumed to be closer, because a blood vessel has a sharper brightness change in the width direction than in the direction of advancement. If necessary, a brightness cross-sectional profile in a diagonal direction may be added in determination of a vessel assessment value.

The CPU 21 proceeds to perform a total adjacent vessel assessment value calculation process (S14), which for each point (pixel) present in the movement range of the puncture needle 13 in the vessel assessment value image 44, determines the total sum of the vessel assessment values for the points on blood vessels that are located within a vicinity region 45 of a predetermined size centered at the point as the puncture suitability, as shown in Figure 9. The reason for determining the puncture suitability in this manner is to prevent direct reflection of local abnormalities of vessel assessment values arising from unevenness and the like into the puncture suitability, which occurs when assessment is made only at a single point within the movement range of the puncture needle 13. Such accidental abnormalities can be statistically eliminated and stable determination can be made by determining the total sum of the vessel assessment values determined for the relevant pixels (points on blood vessels, the same in the description below) within the vicinity region 45 of each pixel (point) of interest as the puncture suitability for that pixel (point) as mentioned above.

However, when the region is simply expanded, sharpness of assessment for each pixel (point) is likely to be lost. Thus, the vessel assessment value for each corresponding pixel (point) in the vicinity region 45 can be weighted in accordance with the distance from the center of the vicinity region 45 to the pixel, such that a higher puncture suitability can be obtained for a pixel for which more points with high vessel assessment values are concentrated around the center.

The CPU 21 then performs a maximum assessment value calculation process (S15), which compares the puncture suitabilities determined for all of the relevant pixels (points) within the movement range of the puncture needle 13 and selects the pixel (point) with the highest suitability as the optimal puncture point.

In selection of the optimal puncture point, it may be simply selected based on the magnitude of the puncture suitability or a further condition related to easiness of puncture may be taken into consideration. For instance, where the body part for puncture is a finger, the skin surface tends to face the puncture needle more easily at a location closer to the center of the finger because a finger has a cylindrical shape. Conversely, toward the edge of the finger, it is more difficult to insert a puncture needle since an angle is more likely to occur relative to the skin surface. Thus, in order to make a central portion of a finger more likely to be chosen, adjustment can be made such that a higher puncture suitability is given to a location closer to a central portion of a finger. Since the position of the finger region in the captured image varies depending on the posture of presenting the finger, the contour of the finger may be extracted by image processing and higher suitability may be given as the location is closer to the central portion of the finger region in the captured image.

(1-3) Effects of the first embodiment

The automatic blood collection apparatus 1 in this embodiment having the above-described configuration can stably determine puncture suitabilities based on vessel assessment values reflecting the thicknesses and densities of blood vessels for certain points on blood vessels within the movement range of the puncture needle 13, automatically select the point with the highest suitability from among them as the optimal puncture point and insert the puncture needle 13 at it. The automatic blood collection apparatus 1 therefore can perform stable blood collection irrespective of the subject's posture of presenting a body part, the condition of the body part, and individual difference in shape or the like.

(2) Second embodiment

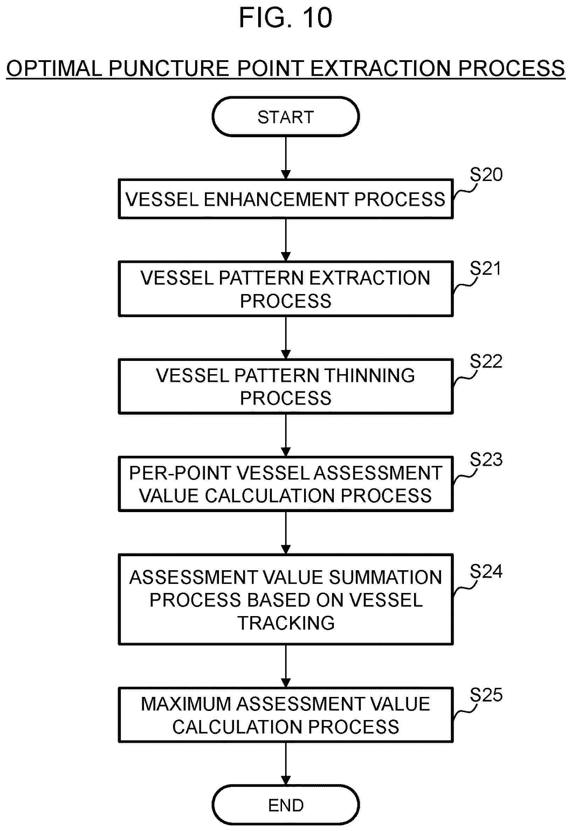

Figure 10 shows a flow of the optimal puncture point extraction process in a second embodiment performed by the CPU 21 of the automatic blood collection apparatus 1 described above for Figures 1 and 2, instead of the optimal puncture point extraction process described with Figure 5.

In this optimal puncture point extraction process, processing in step S24 is different from the processing action in step S14 of the optimal puncture point extraction process in the first embodiment. Processing actions of the other steps, steps S20 to S23 and S25, are similar to those of steps S10 to S13 and S15 of the optimal puncture point extraction process in the first embodiment, respectively.

Specifically, the optimal puncture point extraction process in this embodiment differs from the optimal puncture point extraction process in the first embodiment in that it sums vicinity result assessment values by blood vessel tracking in step S24 instead of determining the total sum of vessel assessment values in the vicinity of a relevant pixel (point).

The method at step S14 of the optimal puncture point extraction process in the first embodiment does not consider continuity of a blood vessel in the vicinity region 45 (Figure 9) in the calculation of puncture suitability. For example, the puncture suitability will indicate high suitability even when dark noises appearing dark, such as blood vessels, occur in many spots in the vicinity of the point for which the puncture suitability is being calculated. Such noise can be easily removed by adding a condition that a blood vessel has continuity.

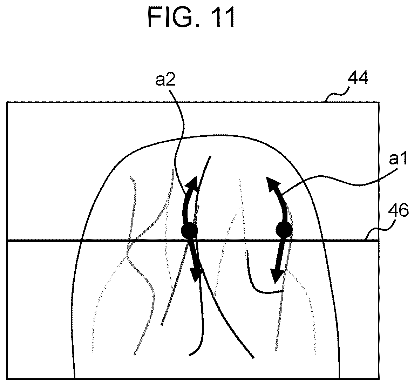

In this embodiment, therefore, at step S24 of the optimal puncture point extraction process, for each point (pixel) on a blood vessel within a movement range 46 of the puncture needle 13, blood vessel tracking is performed (arrows a1, a2 in Figure 11) continuously for a certain distance starting at the point as a starting point as depicted in Figure 11, and the vessel assessment values for the points that are passed through in the tracking are totaled to determine the puncture suitability of that point.

In this manner, rather than mechanically expanding the vicinity region 45 (Figure 9) of the point for which the puncture suitability being calculated as in step S14 of the optimal puncture point extraction process in the first embodiment, a blood vessel running through such points is specifically identified and a range of examination is expanded to its vicinity based on the continuity of the blood vessel. Thus, the puncture suitability can be determined more stably with less influence of the vessel assessment value of a point not connected with the starting point by a blood vessel. In totaling of vessel assessment values, the vessel assessment values for points on a blood vessel may be weighted so that the weight is greater as the tracked distance from the starting point is shorter.

The optimal puncture point extraction process in this embodiment thus can perform more stable blood collection than the optimal puncture point extraction process of the first embodiment.

(3) Third embodiment

In the first and second embodiments, calculation of the puncture suitability for a pixel (point) on a blood vessel in the captured image 40 (Figure 7) of blood vessels was described in connection with an approach that makes estimation based on predefined logical grounds related to vessel characteristics, including the vicinity region of the point. In recent years, recognition techniques based on machine learning that prepare a large amount of learning data and learn patterns from the learning data to make estimation have increasingly been used in practice. Thus, this embodiment also applies machine learning to the calculation of puncture suitability.

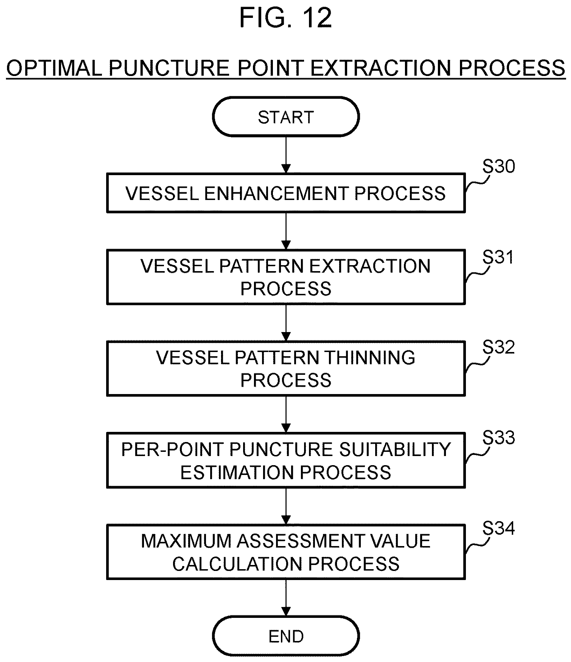

Specifically, in this embodiment, for each point (pixel) on a vessel pattern, the CPU 21 cuts out partial images similar to the vicinity region 45 defined in the total adjacent vessel assessment value calculation process at step S14 or S24 from the vessel enhanced image 41 (Figure 7) which is generated in step S10 of the optimal puncture point extraction process in the first embodiment described with Figure 10 or in step S20 of the optimal puncture point extraction process in the first embodiment described with Figure 11, and labels each partial image with the puncture suitability at the center point thereof, so that the CPU 21 generates in advance a learning model by machine learning using a learning dataset, which is formed of pairs of such a partial image and the puncture suitability at the center point of the partial image, as illustrated in Figure 12. The puncture suitability with which a partial image is labeled is the puncture suitability calculated for the center point of that partial image.

At the time of blood collection, the CPU 21 calculates the puncture suitability for each point (pixel) on a blood vessel in accordance with the processing procedure shown in Figure 12. Figure 12 is a flowchart illustrating a flow of the optimal puncture point extraction process in the third embodiment, which is performed by the CPU 21 of the automatic blood collection apparatus 1 described above with Figures 1 and 2 in place of the optimal puncture point extraction process described for Figure 5.

In practice, in this embodiment, upon proceeding to step S8 of the automatic blood collection process described above for Figure 4, the CPU 21 starts the optimal puncture point extraction process shown in Figure 12 and handles steps S30 to S32 in a similar manner to steps S10 to S12 in Figure 5. Subsequently, for each point (pixel) on a blood vessel within the movement range of the puncture needle 13 in the thinned image 43 (Figure 7) obtained in step S32, the CPU 21 estimates the puncture suitability for that point using the learning model mentioned above (S33). After that, the CPU 21 compares the puncture suitabilities determined for all the points on the blood vessels within the movement range of the puncture needle 13, and selects the point with the highest suitability as the optimal puncture point (S34).

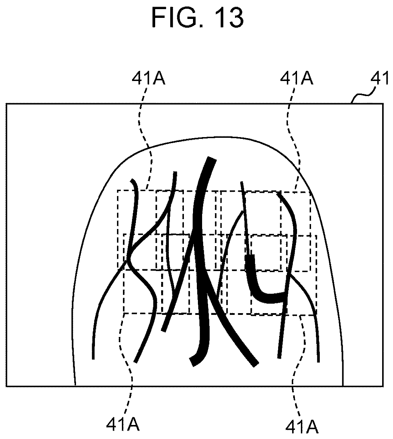

While a great amount of data is required in learning of puncture suitability in order to achieve estimation with high accuracy, many variations of partial images 41A can be cut out with overlaps between them from a single vessel enhanced image 41 as illustrated in Figure 13. If errors or the like occurs using the results of the theoretical calculation method described in the first and second embodiments during labeling of puncture suitability, they may be manually modified where necessary to create a dataset of learning data. This can accelerate building of a dataset of learning data.

The optimal puncture point extraction process in this embodiment thus can perform stable blood collection more speedily than the optimal puncture point extraction process of the first embodiment.

(4) Fourth embodiment

In selection of the optimal puncture point, a point with a remarkably high puncture suitability can exist outside the movement range of the puncture needle. If the subject's cooperation is obtained, the subject can be made to move his/her body part presented at the automatic blood collection apparatus 1 into the movement range of the puncture needle 13, so that the puncture needle 13 can be inserted at a location with higher puncture suitability in the body part and hence more stable blood collection can be performed.

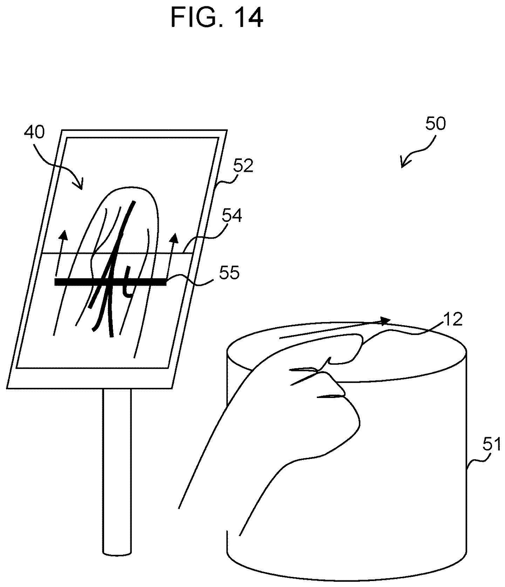

Figure 14 shows a configuration of an automatic blood collection system 50 according to the fourth embodiment, including an automatic blood collection apparatus 51 equipped with the function of prompting the subject to move the body part (here a finger) presented by the subject when necessary. The automatic blood collection system 50 includes a display device 52 and the automatic blood collection apparatus 51. Although Figure 14 describes the display device 52 as a separate device from the automatic blood collection apparatus 51, the automatic blood collection apparatus 51 may include the display device 52.

The display device 52 is formed of a general-purpose display device constructed of a liquid crystal display device, an organic EL (Electro-Luminescence) display device, etc. The display device is coupled to the automatic blood collection apparatus 51 via a cord not shown, and displays an image based on image data provided from the automatic blood collection apparatus 51.

The automatic blood collection apparatus 51 basically has a similar configuration to the automatic blood collection apparatus 1 in the first to third embodiments. A CPU 53 (Figure 1) of the automatic blood collection apparatus 51 in this embodiment also determines the puncture suitability for each of points on blood vessels patterns outside the movement range of the puncture needle 13 in steps S10 to S14 of Figure 5, steps S20 to S24 of Figure 10, or steps S30 to S33 of Figure 12.

The CPU 53 also selects one point (pixel) with the highest suitability in step S15 of Figure 5, step S25 of Figure 10, or step S34 of Figure 12, and if the selected point is not located in the movement range of the puncture needle 13, displays a message or the like on the display device 52 for prompting the subject to move the finger 12 so that the point is located in the movement range of the puncture needle 13.

At this point, a captured image 40 (Figure 7) of the finger 12 being taken by the camera 7, a frame representing the movement range of the puncture needle such as a line 54 or a rectangle indicating the corresponding movement range of the puncture needle 13, and a line (which is called a reference line below) 55 or a frame indicating a point representing the point with the highest suitability selected as discussed above (which is called an out-of-range puncture candidate point below) or a movement range of the puncture needle 13 that passes through the out-of-range puncture candidate point are displayed on the display device 52 while being updated in real time. In this way, the distance between the out-of-range puncture candidate point and the movement range of the puncture needle 13 is visually shown in real time.

On such a display, when the subject moves the finger 12, only the view of the finger 12 moves in the captured image 40 with the movement of the finger 12 by the subject, while the line 54 or frame representing the movement range of the puncture needle 13 shown on the display device 52 remains fixed. In response to the movement, the reference line 55 passing through the out-of-range puncture candidate point also moves in the captured image 40 with the view of the finger 12. This allows the subject to easily recognize in which direction and how much the subject should move the finger 12 based on the line 54 or frame representing the movement range of the puncture needle 13 and the line (reference line) 54 or frame passing through the out-of-range puncture candidate point shown on the display device 52.

The CPU 53 monitors the coordinates of the movement range of the puncture needle 13 and the coordinates of an out-of-range optimal puncture point in real time, and if the movement range of the puncture needle 13 is located in a fingertip direction with respect to the out-of-range puncture candidate point, for example, it displays an instruction message or icon to the effect that the subject should move the finger 12 in the fingertip direction on the display device 52. When the subject moves the finger 12 in response to the message or icon and as a result the out-of-range puncture candidate point overlaps the puncture needle movement range (the out-of-range puncture candidate point has entered the puncture needle movement range), the CPU 53 indicates to the subject that the finger 12 has reached the optimal position with a message or an alarm sound and asks the user to stop the finger at that position. This enables the subject to intuitively place the optimal puncture point within the puncture needle movement range.

As the vessel patterns in the finger 12 are personal information and are information also used in biometric authentication, it can be inappropriate to display a captured image as it is. Accordingly, while the contour of the finger needs to be kept discernible, image processing may be applied for making vessel patterns obscure, such as filling the inside of the finger region with black color.

As described above, the automatic blood collection system in this embodiment can collect a required amount of blood more stably than the automatic blood collection apparatus 1 in the first embodiment.

(5) Other embodiments

In the first to fourth embodiments above, the automatic blood collection apparatuses 1, 51 were described as being configured as shown in Figures 1 and 2. However, the present invention is not limited to this and can apply a wide variety of other configurations.

In the third embodiment, it was described that a learning model is generated by machine learning using a learning dataset that is formed from pairs of a partial image in a vicinity centered at a point on a blood vessel in a captured image and the puncture suitability at the point. However, the present invention is not limited to this; a learning model may be generated by machine learning using a learning dataset that is formed from pairs of a partial image in a vicinity centered at a point on a blood vessel in a captured image and some indicator of suitability equivalent to the puncture suitability at the point.

[Industrial Applicability]

The present invention can be applied to blood collection apparatuses that perform blood collection by inserting a puncture needle into a body part presented by a subject.

[Reference Signs List]

1, 51      automatic blood collection apparatus
2         computation unit
3         light source control unit

| | |
|---|---|
| 4 | image input unit |
| 5 | mechanism control unit |
| 7 | camera |
| 8 | near-infrared light source |
| 9 | movement mechanism |
| 10 | blood collection tube |
| 12 | finger |
| 13 | puncture needle |
| 21, 53 | CPU |
| 40 | captured image |
| 41 | vessel enhanced image |
| 42 | vessel pattern extracted image |
| 43 | thinned image |
| 44 | vessel assessment value image |
| 45 | vicinity region |
| 50 | automatic blood collection system |
| 52 | display device |

**Claims**

1. A blood collection apparatus that performs blood collection by inserting a puncture needle into a body part presented by a subject, the blood collection apparatus comprising:

   a light source which emits light to the body part;
   a camera which captures an image of a blood vessel running subcutaneously in the body part from the same side or the opposite side of the light source;
   a movement mechanism which moves a relative position of the puncture needle to the body part; and
   a computation unit which calculates puncture suitability as a quantitative numerical value for each of points in a range in which the puncture needle is allowed to move on a captured image of the blood vessel captured by the camera, the puncture suitability being an indicator of whether the point is suitable for puncture or not,
   wherein the computation unit selects a location best suited for insertion of the puncture needle in the body part based on the puncture suitability calculated for each point in the range in which the puncture needle is allowed to move on the captured image, and controls the movement mechanism such that the puncture needle is inserted at the selected location.

2. The blood collection apparatus according to claim 1, wherein when a point of interest on the captured image is on the blood vessel, the computation unit calculates the puncture suitability based on a vessel assessment value indicating a value dependent on a thickness and a density of the blood vessel.

3. The blood collection apparatus according to claim 2, wherein the computation unit calculates, as the puncture suitability, a total sum of the vessel assessment values for points on a blood vessel that are located within a vicinity region centered at the point of interest on the captured image.

4. The blood collection apparatus according to claim 3, wherein the computation unit calculates the puncture suitability with weighting of the vessel assessment values for the points within vicinity region according to a distance from a center of the vicinity region.

5. The blood collection apparatus according to claim 2, wherein the computation unit performs tracking along the blood vessel continuously for a distance starting at the point on the blood vessel in the captured image as a starting point, and calculates a total sum of the vessel assessment values for points on the captured image that are passed through in the tracking as the puncture suitability for the point on the blood vessel.

6. The blood collection apparatus according to claim 5, wherein the computation unit calculates the puncture suitability with weighting of the vessel assessment values for the points on the captured image that are passed through in the tracking according to a tracked distance from the starting point.

7. The blood collection apparatus according to claim 1, wherein the computation unit generates a learning model by machine learning using a learning dataset and uses the generated learning model to estimate the puncture suitability for each of the points on the blood vessel in the captured image, the learning dataset being formed of pairs of a partial image in a vicinity centered at a point on a blood vessel in the captured image and the puncture suitability or a suitability indicator equivalent to the puncture suitability at the point.

8. The blood collection apparatus according to claim 1, wherein the computation unit also calculates the puncture suitability for each of points on a blood vessel outside a movement range of the puncture needle in the captured image, and when there is a point with the puncture suitability higher than a highest puncture suitability within the movement range of the puncture needle among the points outside the movement range of the puncture needle, the computation unit gives the subject an instruction to move the body part such that the point is moved into the movement range of the puncture needle.

9. The blood collection apparatus according to claim 8, wherein the computation unit visually displays a direction in which the subject is to move the body part.

10. The blood collection apparatus according to claim 8, wherein when the point that is outside the movement range of the puncture needle and has the puncture suitability higher than the highest puncture suitability within the movement range of the puncture needle has entered the movement range of the puncture needle, the computation unit notifies the subject that the point has entered the movement range of the puncture needle.

11. A method of blood collection performed by a blood collection apparatus that performs blood collection by inserting a puncture needle into a body part presented by a subject, the blood collection apparatus comprising:

a light source which emits light to the body part;
a camera which captures an image of a blood vessel running subcutaneously in the body part from the same side or the opposite side of the light source;
a movement mechanism which moves a relative position of the puncture needle to the body part; and
a computation unit which calculates puncture suitability as a quantitative numerical value for each of points in a range in which the puncture needle is allowed to move on a captured image of the blood vessel captured by the camera, the puncture suitability being an indicator of whether the point is suitable for puncture or not,
the method comprising:

a first step of, by the computation unit, selecting a location best suited for insertion of the puncture needle in the body part based on the puncture suitability calculated for each point in the range in which the puncture needle is allowed to move on the captured image, and
a second step of, by the computation unit, controlling the movement mechanism such that the puncture needle is inserted at the selected location.

# FIG. 1

1(51)

AUTOMATIC BLOOD COLLECTION APPARATUS

COMPUTATION UNIT — 2

21(53)
CPU

22
MEMORY

23
AUXILIARY STORAGE

20

24 I/F

24 I/F

24 24 I/F

24 I/F

4
IMAGE INPUT UNIT

5
MECHANISM CONTROL UNIT

3
LIGHT SOURCE CONTROL UNIT

6
COMMUNI-CATION UNIT

11

8

12

13

9A

9

10

7

## FIG. 2

## FIG. 3

# FIG. 4

## AUTOMATIC BLOOD COLLECTION PROCESS

START

S1
INITIALIZE

S2
INPUT IMAGE

S3
COMPARE CAPTURED IMAGES

S4
FINGER DETECTED? — NO

YES

S5
INDICATE DETECTION OF FINGER

S6
ADJUST LIGHT SOURCE FOR QUANTITY OF LIGHT

S7
ADJUSTMENT TO QUANTITY OF LIGHT COMPLETED? — NO

YES

S8
OPTIMAL PUNCTURE POINT EXTRACTION PROCESS

S9
PUNCTURE MECHANISM CONTROL PROCESS

END

# FIG. 5

## OPTIMAL PUNCTURE POINT EXTRACTION PROCESS

```
        ┌─────────────┐
        │    START     │
        └─────────────┘
               │
               ▼
   ┌───────────────────────────┐
   │ VESSEL ENHANCEMENT PROCESS │  S10
   └───────────────────────────┘
               │
               ▼
   ┌───────────────────────────┐
   │  VESSEL PATTERN EXTRACTION │  S11
   │          PROCESS           │
   └───────────────────────────┘
               │
               ▼
   ┌───────────────────────────┐
   │   VESSEL PATTERN THINNING  │  S12
   │          PROCESS           │
   └───────────────────────────┘
               │
               ▼
   ┌───────────────────────────┐
   │ PER-POINT VESSEL ASSESSMENT│  S13
   │  VALUE CALCULATION PROCESS │
   └───────────────────────────┘
               │
               ▼
   ┌───────────────────────────┐
   │   TOTAL ADJACENT VESSEL    │  S14
   │     ASSESSMENT VALUE       │
   │     CALCULATION PROCESS    │
   └───────────────────────────┘
               │
               ▼
   ┌───────────────────────────┐
   │ MAXIMUM ASSESSMENT VALUE   │  S15
   │    CALCULATION PROCESS     │
   └───────────────────────────┘
               │
               ▼
        ┌─────────────┐
        │     END      │
        └─────────────┘
```

FIG. 6

# FIG. 7

# FIG. 8

BRIGHTNESS
CROSS-SECTIONAL
PROFILE

K1

$W$

CURVATURE
CHANGE RATE

0

K2

# FIG. 9

45    45    44

# FIG. 10

## OPTIMAL PUNCTURE POINT EXTRACTION PROCESS

```
                    ┌─────────────┐
                    │    START    │
                    └─────────────┘
                           │
                           ▼
         ┌──────────────────────────────────┐   S20
         │   VESSEL ENHANCEMENT PROCESS      │
         └──────────────────────────────────┘
                           │
                           ▼
         ┌──────────────────────────────────┐   S21
         │   VESSEL PATTERN EXTRACTION       │
         │           PROCESS                 │
         └──────────────────────────────────┘
                           │
                           ▼
         ┌──────────────────────────────────┐   S22
         │   VESSEL PATTERN THINNING         │
         │           PROCESS                 │
         └──────────────────────────────────┘
                           │
                           ▼
         ┌──────────────────────────────────┐   S23
         │  PER-POINT VESSEL ASSESSMENT      │
         │  VALUE CALCULATION PROCESS        │
         └──────────────────────────────────┘
                           │
                           ▼
         ┌──────────────────────────────────┐   S24
         │  ASSESSMENT VALUE SUMMATION       │
         │  PROCESS BASED ON VESSEL          │
         │           TRACKING                │
         └──────────────────────────────────┘
                           │
                           ▼
         ┌──────────────────────────────────┐   S25
         │  MAXIMUM ASSESSMENT VALUE         │
         │  CALCULATION PROCESS              │
         └──────────────────────────────────┘
                           │
                           ▼
                    ┌─────────────┐
                    │     END     │
                    └─────────────┘
```

# FIG. 11

# FIG. 12

## OPTIMAL PUNCTURE POINT EXTRACTION PROCESS

START

VESSEL ENHANCEMENT PROCESS — S30

VESSEL PATTERN EXTRACTION PROCESS — S31

VESSEL PATTERN THINNING PROCESS — S32

PER-POINT PUNCTURE SUITABILITY ESTIMATION PROCESS — S33

MAXIMUM ASSESSMENT VALUE CALCULATION PROCESS — S34

END

FIG. 13

# FIG. 14

## INTERNATIONAL SEARCH REPORT

| International application No. |
| --- |
| **PCT/JP2024/013271** |

| A. | CLASSIFICATION OF SUBJECT MATTER |
| --- | --- |

*A61B 5/151*(2006.01)i; *G06T 7/00*(2017.01)i
FI:   A61B5/151 100; G06T7/00 612

According to International Patent Classification (IPC) or to both national classification and IPC

| B. | FIELDS SEARCHED |
| --- | --- |

Minimum documentation searched (classification system followed by classification symbols)

A61B5/15-5/151; A61B10/00; A61B5/107; G06T7/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 2020-185199 A (KABUSHIKI KAISHA NIHON MICRONICS) 19 November 2020 (2020-11-19)<br>paragraphs [0011]-[0039], fig. 1-4 | 1-2, 11 |
| A | paragraphs [0011]-[0056], fig. 1-10 | 3-10 |
| Y | JP 8-164124 A (NIKON CORPORATION) 25 June 1996 (1996-06-25)<br>paragraphs [0023], [0028]-[0029], fig. 1-2 | 1-2, 11 |
| A | paragraphs [0020]-[0037], fig. 1-2 | 3-10 |
| A | JP 2019-88391 A (HITACHI HIGH-TECHNOLOGIES CORPORATION) 13 June 2019 (2019-06-13)<br>paragraphs [0021]-[0084], fig. 1-11 | 1-11 |
| A | CN 112381816 A (UNIV TONGJI) 19 February 2021 (2021-02-19)<br>paragraphs [0060]-[0125], fig. 1-7 | 1-11 |

☐ Further documents are listed in the continuation of Box C.      ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **03 June 2024** | **11 June 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT
**Information on patent family members**

International application No.

**PCT/JP2024/013271**

| Patent document cited in search report | | | Publication date (day/month/year) | Patent family member(s) | | | Publication date (day/month/year) |
|---|---|---|---|---|---|---|---|
| JP | 2020-185199 | A | 19 November 2020 | US | 2022/0257874 | A1 | |
| | | | | paragraphs [0021]-[0049], fig. 1-4 | | | |
| | | | | WO | 2020/230408 | A1 | |
| | | | | CN | 113840567 | A | |
| JP | 8-164124 | A | 25 June 1996 | (Family: none) | | | |
| JP | 2019-88391 | A | 13 June 2019 | WO | 2019/093124 | A1 | |
| | | | | paragraphs [0021]-[0084], fig. 1-11 | | | |
| CN | 112381816 | A | 19 February 2021 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6994910 B

**Non-patent literature cited in the description**

- **LI, X.** ; **LIN, J.** ; **PANG, Y.** ; **HUANG, L.** ; **ZHONG, L.** ; **LI, Z.** Fingertip Blood Collection Point Localization Research Based on Infrared Finger Vein Image Segmentation. *IEEE Transactions on Instrumentation and Measurement*, 2021, vol. 71, 1-12